# EUROPEAN PATENT SPECIFICATION

(11) **EP 3 125 976 B1**
(45) Date of publication and mention of the grant of the patent: **12.06.2019**
(21) Application number: 15712983.4
(22) Date of filing: 02.04.2015
(51) Int. Cl.: A61M 15/00, A61M 11/00

(54) **DRY POWDER INHALER**
TROCKENPULVERINHALATOR
INHALATEUR DE POUDRE SÈCHE

(30) Priority: 03.04.2014 SE 1450409
(43) Date of publication of application: 08.02.2017
(73) Proprietor: Iconovo AB, 223 63 Lund (SE)
(72) Inventor: LASTOW, Orest, S-247 45 Torna Hällestad (SE); ARVIDSSON, Lars, S-247 51 Dalby (SE)
(74) Representative: Ström & Gulliksson AB
(86) International application number: PCT/EP2015/057314
(87) International publication number: WO 2015/150517

(56) References cited:
- EP-A1- 0 387 222
- WO-A1-01/39823
- WO-A1-2011/059953
- WO-A1-2011/059968
- WO-A2-2009/145673
- DE-B3-102005 046 645
- DE-C1- 4 415 462
- US-A- 4 805 811
- US-A1- 2003 136 405

## Description

### Field of the Invention

This invention pertains in general to the field of medicament inhalers, and more particularly to dry powder inhalers. The inhaler comprises at least one air inlet and at least one air outlet, and a reservoir for housing a dry powder drug, the inhaler having an airflow from the at least one air inlet to the at least one outlet during inhalation by a user at said at least one outlet, to deliver said dry powder drug during inhalation by the user.

### Background of the Invention

In the pharmaceutical field, with respect to treatment of respiratory and/or other diseases, inhalers have been widely used. Numerous drugs, medications and other substances are inhaled into the lungs for rapid absorption in the blood stream and for local action in the lung with such inhalers.

Inhaled drugs fall into two main categories, in form of liquids, including suspensions, and powders. The choice of category depends on the characteristics of the drugs, medications, etc., to be inhaled.

The most common type of inhaler is the pressurized metered-dose inhaler. In this type of inhaler medication is most commonly stored in solution in a pressurized canister that contains a propellant, although it may also be a suspension. The canister is attached to a plastic, hand-operated actuator. On activation, the metered-dose inhaler releases a fixed dose of medication in aerosol form.

Another kind of inhaler is a nebulizer, which supply medication as an aerosol created from an aqueous formulation.

The kind referred to herein is yet another type, in form of a dry powder inhaler. A dry powder inhaler releases a pre-metered, capsuled, dose or a device-metered dose of powdered medication that is inhaled through the inhaler. Inhalers with device-metered dose of powdered medication is normally inhalers with medication reservoir, containing powdered medication, from which metered doses are withdrawn through the use of different dose metering arrangements, said doses then being inhaled.

Dry powder inhalers need to deliver a particle size that is predominantly below 5 microns, and preferably between 1 micron and 3.3 microns, for maximum effectiveness. Such small particles are, however, very cohesive due to high surface energy. Agglomeration may be worsened by moisture, and when the medication comprises more than one active substance, since the different active substances may have such properties as to form agglomerations with each other or with pharmaceutical carriers etc. Agglomeration of small particles is a problem which results in the active particles leaving the inhaler as large agglomerates.

EP0237507 discloses one such powder inhaler with a device metered dose, comprising a medicament chamber, a dosing mechanism, and a flow path from an air inlet to an air/medicament outlet. In the flow path deflectors are arranged, to increase deaggregation of medicament. However, this device is limited to medicaments having one active substance or active substances that are compatible with each other during storing. Additionally, medicament will accumulate at the deflectors, decreasing uniformity of dosage.

WO2011059968, WO2009145673, DE102005046645, WO0139823, US2003136405, and WO2011059953 discloses prior art inhalers.

In particular, WO2009145673 discloses a dry powder inhaler with the features of the preamble of claim 1.

An additional problem of the prior art inhalers is that they are either suitable for micronized formulations or carrier based formulations - never both or combinations of these.

In view of these drawbacks and limitations of the prior art, what is needed is a dry powder inhaler device in which effective and satisfactory dispersion of the dry powder is obtained, which inhaler can administer medicament comprising substances which are incompatible in mixture, and an inhaler with increased deaggregation and a more uniform dosage, as well as an inhaler which is suitable for both micronized formulations and/or carrier based formulations.

### Summary of the Invention

Accordingly, the present invention preferably seeks to mitigate, alleviate or eliminate one or more of the above-identified deficiencies in the art and disadvantages singly or in any combination and solves at least the above mentioned problems by providing a dry powder drug inhaler according to claim 1. The dry powder inhaler according to the invention has a distal end and a proximal end and comprises: at least one inlet and at least one outlet, wherein a communication between said at least one inlet and said at least one outlet at least comprises a dosage communication in a dose administering position, wherein the at least one outlet is arranged at the proximal end of the dry powder drug inhaler, while the at least one inlet is arranged at a zone in the opposite distal end of the dry powder inhaler, the at least one outlet is arranged centrally, along a longitudinal axis of the dry powder inhaler; a first medicament reservoir for housing a dry powder first medicament; a second medicament reservoir for housing a dry powder second medicament; a dosage mechanism for withdrawing an inhaler metered dose of the dry powder in the first and second medicament reservoirs in a dose collecting position from the reservoirs into said dosage communication in the dose administering position; wherein the dosage mechanism further comprises a dose disc with at least one opening per reservoir, wherein the dose disc may be rotated between the dose collecting position, wherein the openings are positioned in the reservoirs, and the dose administering position, wherein the openings communicate with said dosage communication; wherein the dose disc is configured such that, in the dose administering position, a first set of two openings are superimposed the communications, while a second set of two openings are positioned in the medicament reservoirs, respectively, wherein the first and second set of two openings are comprised in the openings of the dose disc; further comprising one dosage communication per reservoir, said dosage communications extending from one inlet each to a mixing and deaggregation chamber; wherein the dosage communications are S-shaped between the corresponding inlet and the chamber; and further comprising an inhalation chimney interconnecting the chamber and the at least one outlet; wherein the inhalation chimney is directed upwardly towards the proximal end.

According to the invention, a method, for preparing a dose for inhalation, in an inhaler according to the invention, is also provided for the same purpose.

Further advantageous embodiments are disclosed in the dependent patent claims.

### Brief Description of the Drawings

These and other aspects, features and advantages of which the invention is capable, will be apparent and elucidated from the following description of embodiments of the present invention, reference being made to the accompanying drawings, in which
Fig. 1 is a cross sectional view along a longitudinal axis of an inhaler according to one embodiment in the dose administering position of the present invention;
Fig. 2 is a perspective and cross sectional view of an inhaler according to one embodiment of the present invention;
Fig. 3 is a perspective and cross sectional view of an inhaler according to one embodiment of the present invention, focusing on arrangement of openings in dose disc;
Fig. 4 is a perspective and cross sectional view of an inhaler according to one embodiment of the present invention, focusing on arrangement of openings in dose disc;
Fig. 5 is a perspective and cross sectional view of an inhaler according to one embodiment of the present invention, focusing on arrangement of medicament scraper over opening in dose disc;
Fig. 6 is a perspective and cross sectional view of an inhaler according to one embodiment of the present invention, focusing on arrangement of medicament scraper and medicament in opening;
Fig. 7 is a cross sectional view along a longitudinal axis of an inhaler according to an embodiment of the present invention, focusing on arrangement of medicament scraper over opening in dose disc; and
Fig. 8 is a cross sectional view along a longitudinal axis of an inhaler according to one embodiment, wherein the air and air/medicament flows through this inhaler is disclosed.

### Description of embodiments

The following description focuses on an embodiment of the present invention applicable to a medicament inhaler, and in particular to a dry powder drug inhaler with more than one medicament reservoir, such as two medicament containers.

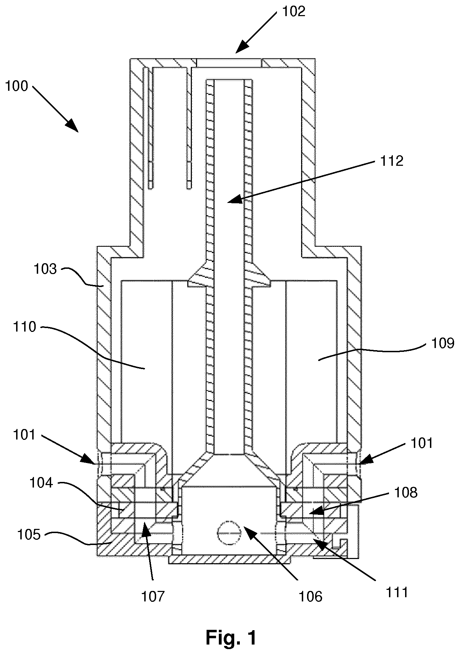

Figs. 1 to 7 illustrate a dry powder drug inhaler 100. The dry powder drug inhaler 100 comprises air inlets 101 and an air outlet 102. The outlet 102 is arranged at a first end of the dry powder drug inhaler 100, while the inlets 101 is arranged at a zone in an opposite second end of the dry powder drug inhaler 100. The outlet 102 is arranged centrally, along the longitudinal axis of the dry powder drug inhaler 100. The inlets 101 may be arranged at a radial, in relation to the longitudinal axis of the dry powder drug inhaler 100, periphery of the dry powder inhaler 100, such that the inlets 101 lead inhaled air transversally and radially towards the central portion of the dry powder inhaler 100.

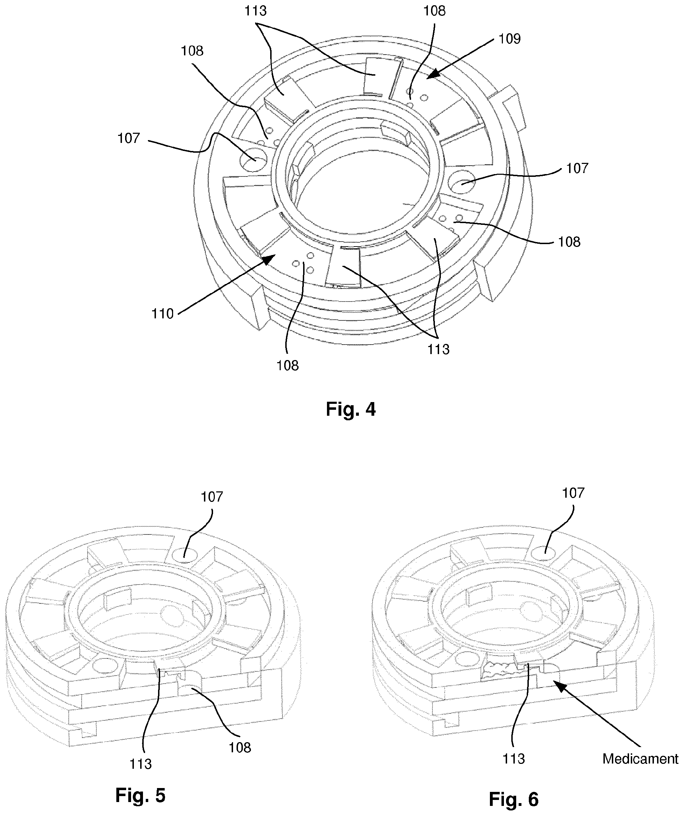

As disclosed in the embodiment according to Fig. 4, which will be further described below, the inlets 101 may however also be positioned with a direction being parallel with the central axis of the dry powder inhaler 100.

The number of inlets and outlets may be different from what is disclosed in Figs. 1 to 4. The number of inlets may for example be adjusted in accordance with needs and specific inhaler design, such that a number of smaller inlets, for reducing pressure fall over the inhaler, are arranged circumferentially of the dry powder inhaler 100. This is not shown. In a corresponding manner the number of air outlets may be adjusted in accordance with needs and specific inhaler design.

The different parts of the dry powder inhaler 100 may be manufactured in a suitable material, such as injection moldable plastics, such as thermoplastics.

The dry powder inhaler 100 comprises three major parts in form of an upper proximal reservoir housing 103, a dose disc 104, and a lower distal twister 105. The reservoir housing 103 and the twister cooperates so as to house the dose disc 104 in between these two. The twister 105 cooperates with the dose disc 104, such that the dose disc 104 may be rotated, via rotation and twisting of the twister 105, between a dose administering position and a dose collecting position. This may be accomplished by interconnecting the dose disc 104 and the twister 105 via interconnecting grooves and ribs, or letting the twister 105 extend longitudinally centrally of the dose disc 104 and connected thereto, such as disclosed for example in Fig. 1. Preferably, the rotation of the dose disc 104 has two end positions, corresponding to the dose administering position and the dose collecting position, in its relation with the reservoir housing 103, in a known manner.

In the dose administering position, the inlets 101 are in fluid communication with a mixing and deaggregation chamber 106 via dosage communications 107. The dosage communications 107 then run through openings 108 in the dose disc 104. Hence, the openings 108, in the dose administering position, are superimposed the communications 107. When rotating the dose disc 104 into a dose collecting position, the openings 108 are rotated away from fluid communication with the inlets 101 and the chamber 106. Instead, the openings 108 are rotated into medicament reservoirs 109, 110, wherein the openings 108 may collect a medicament housed in the reservoirs 109, 110. The medicament contained in the medicament reservoir 109 may be a medicament different from the medicament contained in the medicament reservoir 110. Due to the two reservoirs 109, 110, the inhaler 100 may deliver two substances in one inhalation, said two substances otherwise being incompatible, meaning that these two substances not would be possible to be comprised in one joint reservoir, such that a dry powder inhaler device 100 in which effective and satisfactory dispersion of the dry powder is obtained, which inhaler 100 can administer medicament comprising substances which can be incompatible in mixture or for other reasons are preferred to have in separate reservoirs.

The dose disc 104 and the openings 108 thereof are arranged such that when a first set of two openings 108 are superimposed the communications 107, i.e. in a dose administering position, a second set of two openings 108 are positioned in the medicament reservoirs 109, 110, respectively. Additionally, the distribution of the openings 108 on the dose disc 104 is such that the dose disc may be rotated in one direction only, which means that when the second set of two openings 108 are superimposed the communications 107, the first set of openings 108 are positioned in the medicament reservoirs 109, 110, respectively. It is also possible to rotate the dose disc 104 in a first direction to superimpose openings 108 over communications 107 in dose administering position, and then rotate the dose disc 104 in the opposite direction into the dose collecting position, to thereafter again rotate the dose disc in the first direction into the dose administering position. When the dose disc 104 is rotated in a first direction into the dose administering position and the opposite direction into the dose collecting position, the dose disc 104 may have rotational stops in the dose administering position and the dose collecting position, respectively, to ensure good superimposition over communications 107 and positioning in the medicament reservoirs 109, 110, respectively.

It is also envisioned to provide the inhaler with more than two, such as three, four, five, or six, reservoirs 109, 110, with the same arrangement of inlets, outlets, communications, dose disc, openings etc., within the ambit of the present invention.

In a comparative example not falling within the scope of the invention, the inhaler 100 may be provided with a different dosage mechanism than the one disclosed above, such as for example electrical drive of different parts, and using paddles instead of dose disc 4. However, the use of the dose disc 104 and its cooperation with the twister 105 and the reservoirs 109, 110 allows for a very cost effective solution, while simultaneously ensuring a high dose accuracy and the other benefits disclosed herein.

The dosage communications 107 are S-shaped. The S-shape is such that that the communications 107 starts at inlets 101 and extend downstream (during inhalation) in a central and transversal direction, where after they bend downwards and distally to extend in a longitudinal and distal direction to - in the dose administering position - pass through the openings 108. In this way, when medicament is positioned in the openings 108, the change of air flow direction will increase the turbulence of the air flow, which will facilitate initial deaggregation of the medicament in the openings 108. This ensures that the all the medicament in the openings 108 will follow the air flow in the communications into the chamber 106, and not partly remain in the openings 108. Downstream (again during inhalation) the openings 108, the communications 107 again bend to be directed into a central and transversal direction, to exit into the chamber 106. Distally below the openings 108, when the communications 107 bend centrally and transversally, ledges 111 are formed. These ledges 111 ensures that medicament intended to be held in the openings 108 until inhalation, which medicament may fall down, still may follow the inhaled air into the chamber 106. This means for example that the reservoirs 109, 110 may comprise a dry powder medicament in form of both a dry powder medicament in form of a micronized formulation or a carrier based formulation, or mixtures thereof. The inhaler 100 may then for example comprise a dry powder medicament in form of a micronized formulation in the first reservoir 109 and a dry powder medicament in form of a carrier based formulation in the second reservoir 110.

The directions of the two communications 107 when entering chamber 106 are arranged such that the air flows, and hence medicament flows during inhalation, when the inhaler 100 is in a dose administering position, cross each other or coincide. In this way, the medicament in the medicament flows will physically interact to increase deaggregation of the medicaments, which may increase dose uniformity, since the need for deflectors then is decreased. This feature also adds the possibility to combine or adapt the inhaler 100 for deliverance of micronized formulations and/or carrier based formulation. Of course, it also possible to combine the feature of crossing or coinciding flows from the two communications 107 with deflectors, even though the need thereof is decreased.

Depending on the medicament to be administered, and the formulation thereof, the openings 108 may be more than one opening 108 per communication 107, such as a set of openings 108, as disclosed in Fig. 4. Some medicaments have other aggregation characteristics, making it difficult to retain the medicament as a "plug" in the opening 108. Then, it may be preferable to make several openings 108 with a relatively smaller diameter. Alternatively, this can be to deliver a smaller amount of powder. This feature also adds the possibility to combine or adapt the inhaler 100 for deliverance of micronized formulations and/or carrier based formulation.

During inhalation, the medicament will then follow the air flow through the communications 107, into the chamber 106, wherein the air/medicament streams from the different communications 107 will cross, such that the medicament agglomerates will collide to increase deaggregation, where after a jet stream of finely dispersed medicament and air will continue through an inhalation chimney 112 out of the inhalator 100 through outlet 102, into the lungs of the user. The chimney 112 increases jet formation, allowing for a maintained low aggregation of medicament, hence increasing potential of medicament to reach out far in the lungs of the patient. The chimney is generally tubular, but could optionally be provided with deflectors, to further increase jet creation. Such deflectors could be bumps or spiral-shaped ridges, extending along the length of the chimney from the chamber 106 to the outlet 102. According to the invention, the chimney 112 is directed upwardly; in comparative examples not falling within the scope of the invention, it can just as well be directed downwardly or to the sides, whereby the outlet 102 naturally instead also is positioned at the bottom or on the sides, respectively. Additionally, the chimney 112 does not have to be generally tubular, but could be bent or sinus-shaped, depending on where on the inhaler 100 it is preferred to position the outlet 112. For flow characteristics and dose reliability and maintenance, it is however preferred to have it directed upwardly and generally tubular with optional deflectors. The general shape of the chimney 112 may also be such as to have differences in cross-sectional area, such as cone-shaped. In this way, the flow velocity in the chimney may be regulated, so as to help in deaggregation at chosen parts.

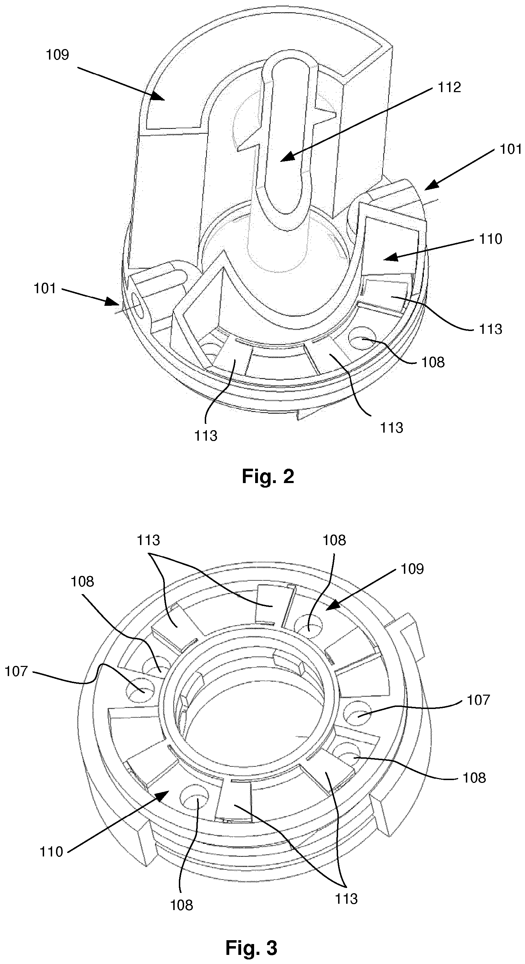

As disclosed in Figs. 2 to 7, the reservoirs 109, 110 could be provided with medicament scrapers 113. The scrapers 113 are suspended at the bottom of the reservoirs 109, 110, such that they bear upon the dose disc 104. The scrapers will pass over the openings 108 of the dose disc 104, so that excessive medicament is removed from the openings 108, to ensure correct dose volume. Also, the scrapers 113 will aid in compacting medicament in the openings 108, which will improve retention of medicament in openings 108 when the dose disc has been rotated into the dose administering position. Since the scrapers 113 are suspended on the reservoirs 109, 110, they will automatically slide along the upper proximal surface of the dose disc 104, when the dose disc 104 is rotated between the dose administering position and dose collecting position. Preferably, each reservoir 109, 110 have a number of scrapers 113 evenly distributed along the bottom of the reservoirs 109, 110. In this way, the scrapers 113 do not only aid in obtaining correct dose volume and dose compacting, but also aid in distributing medicament at the bottom of the reservoirs 109, 110. The number of scrapers 113 per reservoir 109, 110 could for example be selected in the interval of 1 to 6, such as 2 to 4, such as 3. It is also envisioned that the scrapers 113 are arranged in an uneven distribution in the reservoirs 109, 110, if certain reservoirs are configured such that an uneven distribution of the scrapers 113 will have a beneficial effect on the medicament distribution along the bottom of the reservoirs 109, 110.

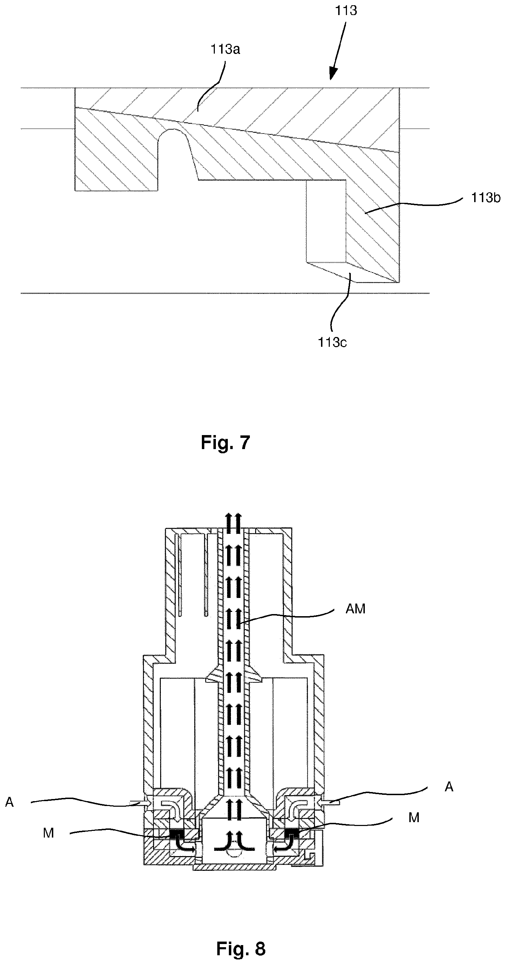

As disclosed in Fig. 7, being a close up cross sectional view of a scraper 113, the scraper 113 could comprise a scraper base portion 113a and a scraper tip portion 113b. The scraper base portion 113a is suspended on the reservoirs 109, 110. The scraper tip portion 113b then extends distally from the scraper base portion 113a. To increase efficiency of the scraper 113 with regard to scraping action on the dose disc 104, the base portion 113a could be manufactured of a resilient material. Such a resilient material could for example be a rubber material. The scraper tip portion 113b is however preferably made of a plastic material, such as a thermoplastic material, of the same kind as the rest of the inhaler 100, such as the dose disc 104, to allow for improved force attributing characteristics and improved interaction with the edge of the opening 108. The scraper tip portion 113b may be provided with a slanted tip 113c. The slanted tip 113c further improves interaction with the edge of the opening 108.

During use, the user will then simply rotate the dose disc 104 in one direction into a dose collecting position if the dose disc is in a dose administering position. Thereafter, the dose disc 104 is rotated preferably into the opposite direction to reach the dose administering position. If the dose disc 104 is already in the dose collecting position, of course the first rotation into the dose collecting position may of course be omitted. During these rotations, the scraper 113 will fill the openings 108 of the dose disc 104 in the reservoirs 109, 110 - the scraper 113 aiding the filling when rotated in both directions. After the dose disc 104 has been rotated into the dose administering position, the openings 108 are filled with medicament - optionally two different medicaments - and in fluid communication with the communications 107. Then the user puts his/her mouth at outlet 102 and inhales. During inhalation air A will enter the inhaler 100 through inlets 101 and flow through communications 107 to carry therewith the medicament(s) M in the openings 108, in accordance with Fig. 8. The air/medicament flow AM will then enter the chamber 106. In the chamber 106, the air/medicament flows AM from the communications will cross each other, such that deaggregation of the medicaments M will increase. Also, the flow characteristics, such as jet stream formation, will increase. Thereafter, the air/medicament flow AM - now comprising air/medicament flows from both communications 107, will go up through the inhaler chimney 112 and out into the lungs of the user through outlet 102.

Although, the present invention has been described above with reference to specific embodiments, it is not intended to be limited to the specific form set forth herein. Rather, the invention is limited only by the accompanying claims.

In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A dry powder inhaler (100) with a distal end and a proximal end comprising:
at least one inlet (101) and at least one outlet (102), wherein a communication between said at least one inlet (101) and said at least one outlet (102) at least comprises a dosage communication (107) in a dose administering position, wherein the at least one outlet (102) is arranged at the proximal end of the dry powder drug inhaler (100), while the at least one inlet (101) is arranged at a zone in the opposite distal end of the dry powder inhaler (100), the at least one outlet (102) is arranged centrally, along a longitudinal axis of the dry powder inhaler (100);
a first medicament reservoir (109) for housing a dry powder first medicament;
a second medicament reservoir (110) for housing a dry powder second medicament;
a dosage mechanism (104, 108) for withdrawing an inhaler metered dose of the dry powder in the first and second medicament reservoirs (109, 110) in a dose collecting position from the reservoirs (109, 110) into said dosage communication (107) in the dose administering position;
wherein the dosage mechanism (104, 108) further comprises a dose disc (104) with at least one opening (108) per reservoir (109, 110), wherein the dose disc (104) may be rotated between the dose collecting position, wherein the openings (108) are positioned in the reservoirs (109, 110), and the dose administering position, wherein the openings (108) communicate with said dosage communication (107);
wherein the dose disc (104) is configured such that, in the dose administering position, a first set of two openings (108) are superimposed the communications (107), while a second set of two openings (108) are positioned in the medicament reservoirs (109, 110), respectively, wherein the first and second set of two openings (108) are comprised in the openings (108) of the dose disc (104);
further comprising one dosage communication (107) per reservoir (109, 110), said dosage communications (107) extending from one inlet (101) each to a mixing and deaggregation chamber (106);
further comprising an inhalation chimney (112) interconnecting the chamber (106) and the at least one outlet (102);
wherein the inhalation chimney (112) is directed upwardly towards the proximal end,
**characterised in that:**
the dosage communications (107) are S-shaped between the corresponding inlet (101) and the chamber (106).

2. The inhaler (100) according to claim 1, wherein the dose disc (104) is provided with rotational stops in the dose administering position and the dose collecting position, respectively.

3. The inhaler (100) according to claim 1, wherein said dosage communications (107) have directions when entering the chamber (106) crossing each other or coinciding with each other.

4. The inhaler according to claim 1 or 2, wherein the dosage communications (107) each comprise a ledge (111) distally of respective opening (108) in the dose administering position.

5. The inhaler (100) according to any of claims 1, 3, or 4, comprising more than one opening (108) per dosage communication (107).

6. The inhaler (100) according to claim 1, wherein the chimney (112) comprises deflectors.

7. The inhaler (100) according to claim 1 or 2, wherein at least one medicament scraper (113) is suspended in each reservoir (109, 110), such that the scraper (113) bears upon the dose disc (104).

8. The inhaler (100) according to claim 7, wherein the number of scrapers (113) per reservoir (109, 110) is selected in the interval from 1 to 6.

9. The inhaler (100) according to claim 7 or 8, wherein the at least one medicament scraper (113) comprises a scraper base portion (113a) and a scraper tip portion (113b).

10. The inhaler (100) according to claim 9, wherein the scraper base portion (113a) is of a resilient material.

11. The inhaler (100) according to claim 10, wherein the resilient material is a rubber material.

12. The inhaler (100) according to any of the preceding claims, wherein the first (109) and second reservoir (110) comprise a dry powder medicament in form of a micronized formulation or a carrier based formulation, or mixtures thereof.

13. The inhaler (100) according to any of the preceding claims, wherein the first reservoir (109) comprises a dry powder medicament in form of a micronized formulation and the second reservoir (110) comprises a dry powder medicament in form of a carrier based formulation.

14. A method, for preparing a dose for inhalation, in the inhaler (100) of any one of claims 1 to 13, comprising the steps of:
providing a dry powder first medicament in the first reservoir (109) of the inhaler (100);
providing a dry powder second medicament in the second reservoir (110) of the inhaler (100);
activating the dosage mechanism (104, 108) of the inhaler (100) to withdraw a dose of said first medicament and a dose of said second medicament into the corresponding dosage communication (107) of the inhaler (100) between the corresponding inlet (101) and the at least one outlet (102) of said inhaler (100)

15. The method according to claim 14, wherein the first medicament is the same or different from the second medicament.

## Patentansprüche

1. Ein Trockenpulverinhalator (100) mit einem distalen Ende und einem proximalen Ende, mit:
zumindest einem Einlass (101) und zumindest einem Auslass (102), wobei eine Verbindung zwischen dem zumindest einen Einlass (101) und dem zumindest einen Auslass (102) zumindest eine Dosierungsverbindung (107) in einer Dosierungsverabreichungsposition aufweist, wobei der zumindest eine Auslass (102) an dem proximalen Ende des Trockenpulvermedikamentinhalators (100) angeordnet ist, während der zumindest eine Einlass (101) an einer Zone in dem entgegengesetzten distalen Ende des Trockenpulverinhalators (100) angeordnet ist, wobei der zumindest eine Auslass (102) zentral entlang einer Längsachse des Trockenpulverinhalators (100) angeordnet ist,
einem ersten Medikamentenreservoir (109) zum Aufnehmen eines ersten Trockenpulvermedikaments,
einem zweiten Medikamentenreservoir (110) zum Aufnehmen eines zweiten Trockenpulvermedikaments,
einem Dosierungsmechanismus (104, 108) zum Abziehen einer Inhalator-abgemessenen Dosis des Trockenpulvers in den ersten und zweiten Medikamentenreservoirs (109, 110) in einer Dosierungssammelposition von den Reservoirs (109, 110) in die Dosierungsverbindung (107) in der Dosierungsverabreichungsposition,
wobei der Dosierungsmechanismus (104, 108) ferner eine Dosierungsscheibe (104) mit zumindest einer Öffnung (108) pro Reservoir (109, 110) aufweist, wobei die Dosierungsscheibe (104) zwischen der Dosierungssammelposition, wo die Öffnungen (108) in den Reservoirs (109, 110) positioniert sind, und der Dosierungsverabreichungsposition, wo die Öffnungen (108) mit den Dosierungsverbindungen (107) kommunizieren, gedreht werden kann,
wobei die Dosierungsscheibe (107) so konfiguriert ist, dass, in der Dosierungsverabreichungsposition, ein erster Satz von zwei Öffnungen (108) den Verbindungen (107) überlagert sind, während ein zweiter Satz von zwei Öffnungen (108) jeweils in den Medikamentenreservoirs (109, 110) positioniert sind, wobei die ersten und zweiten Sätze von zwei Öffnungen (108) in den Öffnungen (108) der Dosierungsscheibe (104) enthalten sind,
ferner mit einer Dosierungsverbindung (107) pro Reservoir (109,110), wobei die Dosierungsverbindungen (107) sich von einem Einlass (101) jeweils zu einer Misch- und Deaggregationskammer (106) erstrecken,
ferner mit einem Inhalationsabzug (112), der die Kammer (106) und den zumindest einen Auslass (102) miteinander verbindet,
wobei der Inhalationsabzug (112) nach oben zu dem proximalen Ende gerichtet ist,
**dadurch gekennzeichnet, dass**:
die Dosierungsverbindungen (107) zwischen dem entsprechenden Einlass (101) und der Kammer (106) S-förmig sind.

2. Der Inhalator (100) gemäß Anspruch 1, wobei die Dosierungsscheibe (104) jeweils mit Rotationsstopps in der Dosierungsverabreichungsposition und der Dosierungssammelposition versehen ist.

3. Der Inhalator (100) gemäß Anspruch 1, wobei die Dosierungsverbindungen (107) Richtungen haben, wenn sie in die Kammer (106) eintreten, die einander kreuzen oder miteinander übereinstimmen.

4. Der Inhalator (100) gemäß Anspruch 1 oder 2, wobei die Dosierungsverbindungen (107) jeweils einen Absatz (111) distal der jeweiligen Öffnung (108) in der Dosierungsverabreichungsposition aufweisen.

5. Der Inhalator (100) gemäß einem der Ansprüche 1, 3 oder 4, mit mehr als einer Öffnung (108) pro Dosierungsverbindung (107) .

6. Der Inhalator (100) gemäß Anspruch 1, wobei der Abzug (112) Ablenkelemente aufweist.

7. Der Inhalator (100) gemäß Anspruch 1 oder 2, wobei zumindest ein Medikamentenschaber oder -abstreifer (103) in jedem Reservoir (109, 110) so aufgehängt ist, dass der Schaber oder Abstreifer (103) an der Dosierungsscheibe (104) anliegt.

8. Der Inhalator (100) gemäß Anspruch 7, wobei die Anzahl von Schabern oder Abstreifern (113) pro Reservoir (109, 110) in dem Intervall von 1 bis 6 ausgewählt ist.

9. Der Inhalator (100) gemäß Anspruch 7 oder 8, wobei der zumindest eine Medikamentenschaber oder -abstreifer (113) einen Schaber-Basisabschnitt (113a) und einen Schaber-Außenendabschnitt (113b) aufweist.

10. Der Inhalator (100) gemäß Anspruch 9, wobei der Schaber-Basisabschnitt (113a) aus einem elastischen Material gemacht ist.

11. Der Inhalator (100) gemäß Anspruch 10, wobei das elastische Material ein Gummimaterial ist.

12. Der Inhalator (100) gemäß einem der vorstehenden Ansprüche, wobei das erste (109) und das zweite Reservoir (110) ein Trockenpulvermedikament in Form einer mikronisierten Rezeptur oder einer trägerbasierten Rezeptur oder von Mischungen davon aufweist.

13. Der Inhalator (100) gemäß einem der vorstehenden Ansprüche, wobei das erste Reservoir (109) ein Trockenpulvermedikament in Form einer mikronisierten Rezeptur und das zweite Reservoir (110) ein Trockenpulvermedikament in Form einer trägerbasierten Rezeptur aufweist.

14. Ein Verfahren zum Herrichten einer Dosis für eine Inhalation, in dem Inhalator (100) gemäß einem der Ansprüche 1 bis 13, mit den Schritten:
Vorsehen eines ersten Trockenpulvermedikaments in dem ersten Reservoir (109) des Inhalators (100),
Vorsehen eines zweiten Trockenpulvermedikaments in dem zweiten Reservoir (110) des Inhalators (100),
Aktivieren des Dosierungsmechanismus (104, 108) des Inhalators (100), um eine Dosis des ersten Medikaments und eine Dosis des zweiten Medikaments in die entsprechende Dosierungsverbindung (107) des Inhalators (100) zwischen dem entsprechenden Einlass (101) und dem zumindest einen Auslass (102) des Inhalators (100) abzuziehen.

15. Das Verfahren gemäß Anspruch 14, wobei das erste Medikament dasselbe Medikament wie das zweite Medikament ist oder davon unterschiedlich ist.

## Revendications

1. Inhalateur de poudre sèche (100) avec une extrémité distale et une extrémité proximale comprenant :
au moins un orifice d'entrée (101) et au moins un orifice de sortie (102), dans lequel une communication entre ledit au moins un orifice d'entrée (101) et ledit au moins un orifice de sortie (102) comprend au moins un conduit de dosage (107) dans une position d'administration de dose, dans lequel l'au moins un orifice de sortie (102) est agencé au niveau de l'extrémité proximale de l'inhalateur de médicament en poudre sèche (100), tandis que l'au moins un orifice d'entrée (101) est agencé au niveau d'une zone dans l'extrémité distale opposée de l'inhalateur de poudre sèche (100), l'au moins un orifice de sortie (102) est agencé de manière centrale, le long d'un axe longitudinal de l'inhalateur de poudre sèche (100) ;
un premier réservoir de médicament (109) permettant de contenir un premier médicament en poudre sèche ;
un second réservoir de médicament (110) permettant de contenir un second médicament en poudre sèche ;
un mécanisme de dosage (104, 108) permettant de retirer une dose mesurée par inhalateur de la poudre sèche dans les premier et second réservoirs de médicament (109, 110) dans une position de collecte de dose à partir des réservoirs (109, 110) vers ledit conduit de dosage (107) dans la position d'administration de dose ;
dans lequel le mécanisme de dosage (104, 108) comprend en outre un disque de dosage (104) avec au moins une ouverture (108) par réservoir (109, 110), dans lequel le disque de dosage (104) peut être pivoté entre la position de collecte de dose, dans laquelle les ouvertures (108) sont positionnées dans les réservoirs (109, 110), et la position d'administration de dose, dans laquelle les ouvertures (108) communiquent avec ledit conduit de dosage (107) ;
dans lequel le disque de dosage (104) est configuré de sorte que, dans la position d'administration de dose, un premier ensemble de deux ouvertures (108) soit superposé aux conduits (107), tandis qu'un second ensemble de deux ouvertures (108) est positionné dans les réservoirs de médicament (109, 110), respectivement, dans lequel les premier et second ensembles de deux ouvertures (108) sont compris dans les ouvertures (108) du disque de dosage (104) ;
comprenant en outre une communication de dosage (107) par réservoir (109, 110), lesdits conduits de dosage (107) s'étendant depuis un orifice d'entrée (101) chacune jusqu'à une chambre de mélange et de désagrégation (106) ;
comprenant en outre une cheminée d'inhalation (112) interconnectant la chambre (106) et l'au moins un orifice de sortie (102) ;
dans lequel la cheminée d'inhalation (112) est dirigée vers le haut vers l'extrémité proximale,
**caractérisé en ce que** :
les conduits de dosage (107) sont en forme de S entre l'orifice d'entrée (101) correspondant et la chambre (106).

2. Inhalateur (100) selon la revendication 1, dans lequel le disque de dosage (104) est doté de butées rotatives dans la position d'administration de dose et la position de collecte de dose, respectivement.

3. Inhalateur (100) selon la revendication 1, dans lequel lesdits conduits de dosage (107) ont des directions lors de l'entrée dans la chambre (106) se croisant les unes les autres ou coïncidant les unes avec les autres.

4. Inhalateur selon la revendication 1 ou 2, dans lequel les conduits de dosage (107) comprennent chacun un rebord (111) distalement par rapport à une ouverture (108) respective dans la position d'administration de dose.

5. Inhalateur (100) selon l'une quelconque des revendications 1, 3 ou 4, comprenant plus d'une ouverture (108) par conduit de dosage (107).

6. Inhalateur (100) selon la revendication 1, dans lequel la cheminée (112) comprend des déflecteurs.

7. Inhalateur (100) selon la revendication 1 ou 2, dans lequel au moins un racloir à médicament (113) est suspendu dans chaque réservoir (109, 110), de sorte que le racloir (113) repose sur le disque de dosage (104).

8. Inhalateur (100) selon la revendication 7, dans lequel le nombre de racloirs (113) par réservoir (109, 110) est sélectionné dans l'intervalle de 1 à 6.

9. Inhalateur (100) selon la revendication 7 ou 8, dans lequel l'au moins un racloir à médicament (113) comprend une partie de base de racloir (113a) et une partie de bout de racloir (113b).

10. Inhalateur (100) selon la revendication 9, dans lequel la partie de base de racloir (113a) est en matériau élastique.

11. Inhalateur (100) selon la revendication 10, dans lequel le matériau élastique est un matériau de caoutchouc.

12. Inhalateur (100) selon l'une quelconque des revendications précédentes, dans lequel le premier (109) et le second réservoir (110) comprennent un médicament en poudre sèche sous la forme d'une formulation micronisée ou d'une formulation à base de vecteur, ou de mélanges de celles-ci.

13. Inhalateur (100) selon l'une quelconque des revendications précédentes, dans lequel le premier réservoir (109) comprend un médicament en poudre sèche sous la forme d'une formulation micronisée et le second réservoir (110) comprend un médicament en poudre sèche sous la forme d'une formulation à base de vecteur.

14. Procédé, pour la préparation d'une dose pour une inhalation, dans l'inhalateur (100) selon l'une quelconque des revendications 1 à 13, comprenant les étapes de :
fourniture d'un premier médicament en poudre sèche dans le premier réservoir (109) de l'inhalateur (100) ;
fourniture d'un second médicament en poudre sèche dans le second réservoir (110) de l'inhalateur (100) ;
activation du mécanisme de dosage (104, 108) de l'inhalateur (100) pour retirer une dose dudit premier médicament et une dose dudit second médicament dans le conduit de dosage (107) correspondante de l'inhalateur (100) entre l'orifice d'entrée (101) correspondant et l'au moins un orifice de sortie (102) dudit inhalateur (100) .

15. Procédé selon la revendication 14, dans lequel le premier médicament est identique au ou différent du second médicament.
